# EUROPEAN PATENT APPLICATION

(11) **EP 3 982 125 A1**
(43) Date of publication of application: **13.04.2022**
(21) Application number: 20201364.5
(22) Date of filing: 12.10.2020
(51) Int. Cl.: G01N 35/00, G01N 33/84

(54) **SUPPORTING A MEASUREMENT OF A LIQUID SAMPLE**

(71) Applicant: EXIAS Medical GmbH, 8020 Graz (AT)
(72) Inventor: Schlaminger, Michael, 8010 Graz (AT); Kiefer, Emanuel, 8010 Graz (AT); Hindinger, Josef, 8010 Graz (AT)
(74) Representative: Dilg, Haeusler, Schindelmann Patentanwaltsgesellschaft mbH

(57) **Abstract**

It is provided an arrangement (100) and method for supporting a measurement of a liquid sample (103) contained in a container (101), the arrangement comprising: a measurement support equipment (121,127); a camera system (113) adapted to acquire images of a region of interest (115) in which the container is arrangeable; and a processor (119) adapted: to analyze at least one image of the images, in order to obtain an analysis result; and to control the measurement support equipment (121,127) based on the analysis result, wherein the measurement support equipment is in particular controlled without requiring touching anything except the container.

## Description

### Field of invention

The present invention relates to an arrangement and to a method for supporting a measurement of a liquid sample contained in a container. Furthermore, a measuring apparatus is provided which comprises the arrangement for supporting the measurement of the liquid sample.

### Art Background

Measurement systems for measuring a liquid sample, in particular a blood sample, are conventionally known, wherein for example analytes, such as blood gases, electrolytes and metabolites can be measured. Thereby, the liquid sample is introduced into a measurement chamber comprising one or more sensitive areas for measuring different constituents of the sample.

Conventionally, measuring a sample is divided in a pre-analytic phase, an analytic phase and a post-analytic phase. Basis for a precise and reliable laboratory diagnostics is an optimal pre-analytic phase. In this pre-analytic phase, the appropriate examinations are selected and the sample is taken, for example from a patient. Furthermore, the sample potentially needs to be prepared and stored in an appropriate manner. During all of these sub-steps of the pre-analytic phase disturbances or errors may occur which may significantly deteriorate the final measuring result. For minimizing such errors, substantial effort is necessary. The samples for example need to be examined whether they comply with predetermined criteria associated with the intended examination. For example, it must be assured that the sample is unambiguously labeled. Further it needs to be checked whether the sample satisfies requirements for performing the measurement. Furthermore, also the container in which the sample is contained potentially needs to satisfy several requirements. For example, all blood examinations are strongly regulated and need to comply with a number of regulations and guidelines.

Conventionally, the detection of pre-analytic errors is in the realm of the user, for example medical personnel. For preventing or detecting of errors in the pre-analytic phase respective guidelines are imposed for laboratory personnel. However, despite of such guidelines, the compliance with these guidelines may not be assured. Furthermore, detecting errors may not be guaranteed. Conventional solutions for decreasing the error rate are often cost-intensive and require user interaction.

Conventionally, measurement equipment requires user interaction at the apparatus via a corresponding input device, such as a touchscreen, keyboard, bar code reader. However, every user interaction requires time and money and is potentially erroneous.

Therefore, there is the constant wish to develop new technologies in order to simplify the handling of the measurement apparatuses and the handling of the samples. Furthermore, new technologies are developed in order to detect the occurrence of errors in the pre-analytic phase. Furthermore, healthcare budgets are under permanent pressure to save expenses and simultaneously improve efficiency and quality. Gain of efficiency may in particular be achieved in that the error rate is decreased and the layout and the logistics of the laboratory is improved.

It has been reported that the pre-analytic phase is more error prone than for example the analytic phase. For example, sample hemolysis and/or insufficient sample volume are examples of often occurring errors during the pre-analytic phase. Further errors relate to erroneous data transcription, tests without quality control, not registered critical values and considerably delayed results.

Another issue to be considered in laboratory diagnostics is that the sample is potentially infectious, for example when blood is intended to be analyzed. During handling of the sample contamination of surfaces within the laboratory cannot be excluded. Therefore, every touch with contaminated surfaces presents a risk for the medical personnel. The same holds for input equipment for introducing the sample into the measurement chamber.

An object of the present invention may be regarded as providing an arrangement for supporting a measurement of a liquid sample, in order to improve at least some steps during the pre-analytic phase of a measurement of a liquid sample. In particular, the object may include to reduce or even avoid errors in handling the sample, to reduce errors regarding the type and constitution of the sample, to reduce errors regarding sample identification and further to reduce the risk of contamination of a user performing the different phases of the examination of the sample. An object may also be regarded as reducing errors conventionally occurring during the pre-analytic phase of a measurement of a liquid sample.

### Summary of the Invention

The object is solved by the subject-matter of the independent claims being directed to an arrangement and a method, respectively, for supporting a measurement of a liquid sample contained in a container. Furthermore, a measuring apparatus is provided which comprises the arrangement for supporting the measurement of the liquid sample. The dependent claims specify particular embodiments of the present invention.

Thereby, it should be understood that features which are described, explained, provided or applied to an arrangement for supporting a measurement of a liquid sample are also, individually or in any combination, applicable to a method of supporting a measurement of a liquid sample according to embodiments of the present invention and vice versa.

According to an embodiment of the present invention it is provided an arrangement for supporting a measurement of a liquid sample contained in a container, the arrangement comprising: a measurement support equipment; a detection system adapted to acquire object information of an object in a region of interest in which the container is arrangeable; and a processor adapted: to analyze the object information, in order to obtain an analysis result; and to control the measurement support equipment based on the analysis result, wherein the measurement support equipment is in particular controlled without requiring touching anything except the container.

The detection system may utilize electromagnetic radiation, such as RADAR, laser scanning, one or more cameras sensitive for visible light and/or infrared light and/or ultraviolet light for detecting the object. The detection system may utilize an ultrasonic sound sensor and/or generator. A combination of different techniques can be provided.

The liquid sample may for example be any kind of biological sample, such as a blood sample of a human being. The liquid sample may however also comprise or be a quality control solution or a calibration solution for ensuring quality control and/or calibration of a measurement apparatus which is supported by the arrangement.

The actual measurement may relate to a measuring of the concentration of at least one analyte. For each particular analyte to be measured, at least one separate sensitive area may be provided within a measurement chamber of the measurement apparatus. The measurement apparatus may for example be configured to perform potentiometric and/or amperometric measurement and/or conductometric measurements, i.e. measurements of electrical potentials and/or electric currents and/or electric impedances. Therefore, within the measurement chamber at least one electrode may be arranged. Other embodiments may alternatively and/or additionally comprise other sensors, such as optical measurements systems.

The arrangement for supporting the measurement of the liquid sample in particular supports the pre-analytic phase or at least one step of this pre-analytic phase. Thereby, handling of the sample by the user may be simplified, the risk for the user to get contaminated may be reduced and the compliance of the sample with the intended measurement may be monitored and ensured.

Thereby, the arrangement may for example determine the characteristics of the sample, for example regarding amount of volume, homogeneity, occurring bubbles, density and so forth. If the characteristic of the sample does not comply with the intended measurement, the arrangement may indicate an error message to the user.

The measurement support equipment may be configured in different manners, as will be explained in detail below. The measurement support equipment is controlled depending on the analysis result as determined by the processor by analyzing at least one image of the images.

The region of interest is in the field of view of the detection system. The region of interest may for example be a region close to an input port of a measuring apparatus, wherein the input port may allow loading the sample into a measurement chamber of the measurement apparatus. Thereby, the arrangement is appropriately configured for supporting in particular loading and/or unloading the sample into the measurement chamber of the measurement apparatus.

The container may be held by a user, such as medical personnel, and may be moved by hand into the region of interest. In other embodiments, also an additional auxiliary equipment may be utilized for traversing or moving the container into the region of interest.

When the container is within the region of interest, it is also in the field of view of the detection system such that the detection system may acquire information about the container in which the liquid sample is contained. The processor may recognize the container by processing the object information. The processor may in particular detect, by analyzing the objection information, at least a type of the container and/or a characteristics of the sample. Furthermore, the processor may detect one or more labels on the container or for example on a container cap. The processor may also analyze the objection information in regard of compliance or non-compliance with an intended measurement to be performed later. If the processor concludes that a compliance with an intended later measurement is satisfied, the processor may control the measurement support equipment to adopt a configuration in which the sample may be loaded (into the measurement chamber). In case non-compliance is concluded by the processor, the measurement support equipment may be controlled to indicate a warning message or an error message to the user, in order for example to change the sample, to change the container or for example to select another measurement procedure.

Thus, controlling the measurement support equipment may e.g. comprise to indicate some information to the user and/or change position and/or orientation of some loading equipment, depending on the analysis result.

The arrangement may support a process in which the user, for example the medical personnel, does not need to touch anything of the arrangement or a measurement apparatus. The user at most may hold the container and may move the container into the region of interest. However, even this is not necessary in other embodiments, when the container is moved by for example a robotic device or some other traversing equipment into the region of interest. When the user does not need to touch anything of the arrangement or a measurement apparatus, a risk of contamination of the user is reduced or even avoided.

Furthermore, since the processor may be capable of determining whether the container and/or the sample satisfies the requirements of an intended examination, also erroneous measurement of a known compatible sample or a known compatible container may be avoided. Thus, at least several steps in the pre-analytic phase of measurement of a liquid sample may be improved.

According to an embodiment of the present invention, the detection system comprises: a camera system adapted to acquire images of the region of interest as at least a part of the object information, wherein the processor is adapted to analyze at least one image of the images, in order to obtain at least partly the analysis result.

The camera system may comprise one or more cameras, such as CCD cameras, CMOS cameras or the like. Each camera may comprise an array of light-sensitive elements, such as photo-diodes, CCD cells or CMOS cells. The light-sensitive areas may be sensitive to visible light and/or to UV-light and/or infrared light. Each camera may comprise one or more lenses. The camera system may be adapted to continuously acquire images of the region of interest and those images may continuously be analyzed by the processor, in particular in real-time. The camera system may be adapted to acquire gray images, or color images.

The region of interest is in the field of view of the camera system. When the container is within the region of interest, it is also in the field of view of the camera system such that the camera system may acquire images containing the container in which the liquid sample is contained. The container may be imaged in different orientations. The processor may recognize the container by image-processing including object detection. The processor may execute image processing software which may have been trained to recognize in the acquired images one or more object types including different types of containers even when imaged in different orientations.

The processor may in particular detect, by analyzing the image, at least a type of the container and/or a characteristics of the sample. Furthermore, the processor may detect (in the image(s)) one or more labels on the container or for example on a container cap. The processor may also analyze the image in regard of compliance or non-compliance with an intended measurement to be performed later.

According to an embodiment of the present invention, the measurement support equipment comprises at least one of: an optical and/or acoustical output device, in particular a display; a sample input system adapted to allow input of the sample for the measurement, the sample input system in particular including at least one of: a sample input opening; a sample aspirator including a sample aspirator tube, in particular needle, having a lumen through which the sample can be conveyed towards a measurement chamber; a sample aspirator positioner configured to adjust the sample aspirator tube in position and/or orientation; a sample input port closure configured to open and close an input port through which the sample aspirator tube is traversable to be accessible in the region of interest.

Thereby, the measurement may be supported by different equipment configured for indicating some information to the user and/or for facilitating loading and/or unloading of the sample and/or protecting sensitive areas within a measurement apparatus. Thereby several steps in the pre-analytic phase may be appropriately supported.

The sample input system may allow to input the sample from a front and/or a side. The sample input opening may allow to connect e.g. a tip of a syringe, e.g. by a Luer lock.

For aspirating or loading the sample the container may be held by the user or it may be held by holding equipment and/or at least partly drawn into a loading region partly within the measurement apparatus.

The optical and/or acoustical output device may be configured to output an optical indication and/or an acoustical indication to a user. Therefore, an optical output device may for example comprise a display and/or a projector and a projection surface. The acoustical output device may for example comprise a loudspeaker.

The sample aspirator may be provided for aspirating the sample into a measurement chamber of a measurement apparatus of which the arrangement for supporting the measurement may be a part. The sample aspirator tube may for example be manufactured from ceramics, glass or metal. The sample aspirator tube may be a straight tube. The sample aspirator may comprise an aspirating unit, such as a pump, for example a peristaltic pump in order to exert or apply a sucking force to the sample aspirator tube to partly evacuate the lumen of the tube or apply underpressure. When the tip of the sample aspirator tube is immersed into the sample, i.e. arranged to be below the surface of the sample, the sample may be drawn into the lumen and from there into the measurement chamber. The processor may be adapted to control the suction force or pressure and/or the speed of the sample as running through the lumen of the sample aspirator tube. The processor may also be adapted to expel the sample or a washing solution or any other solution out of the lumen of the sample aspirator tube by applying an excess pressure (overpressure) to the lumen of the tube or an excess pressure to a sample measurement space. By applying an underpressure to the lumen, the sample may be sucked into the lumen.

The sample aspirator positioner may allow to appropriately position the sample aspirator tube. Thereby it may be allowed to orient or move the sample aspirator tube such that the example may be loaded from the respective container. For example, depending on a detected type of the container, the processor may control the sample aspirator positioner to position and/or orient the sample aspirator tube in positions/orientations which depend on the detected container type.

The sample input port closure (not necessarily present) may be a portion of a measurement apparatus. Through the sample input port the sample aspirator tube may be traversed to be accessible (in particular the tip thereof) in the region of interest. The sample input port closure may protect a measurement chamber or other components of the measurement apparatus.

It should be understood that the optical and/or acoustical output device and/or the sample aspirator and/or the sample aspirator positioner and/or the sample input port closure may or may not be part of the arrangement for supporting a measurement of a liquid sample. However, the processor may at least be configured to generate (or supply) respective control signals to those elements or devices for controlling the behavior of those devices. In other embodiments those examples of the measurement support equipment may be part of the arrangement.

According to an embodiment of the present invention, to control the measurement support equipment based on the analysis result comprises at least one of: to control the sample input system; to indicate information on the optical and/or acoustical output device, in particular to display information on the display; to actuate the sample aspirator; to actuate the sample aspirator positioner; to actuate the sample input port closure.

For controlling the measurement support equipment the processor may generate appropriate control signals or may in particular at least generate respective triggering signals. For example, triggering signals may be sent to respective driver circuits which may be provided for for example the output device, the sample aspirator, the sample aspirator positioner, the sample input port closure.

According to an embodiment of the present invention, to analyze the at least one image includes, in particular in real time: object detection, in particular using methods of unsupervised and/or supervised machine learning (e.g. applying a neural network) configured to identify in the image at least one object type from a group of candidate object types; wherein the measurement support equipment is controlled based on the identified object type.

Object detection may for example employ correlation techniques to correlate acquired images with pre-stored reference-images showing different types of containers (in different orientations) for example. The object detection may for example comprise container type detection and/or sample type detection and/or sample characteristic detection and so forth. Depending on the intended measurement process to be performed later, the object detection may be adapted in order to detect or determine all characteristics of the container and/or the sample which need to comply with predetermined requirements depending on the intended measurement procedure to be performed later. Thereby compliance or non-compliance with an intended measurement may reliably be determined for supporting the measurement.

According to an embodiment of the present invention, to analyze the at least one image includes, in particular in real time, at least one of: detecting a type of the container, in particular syringe or sample tube or capillary or sampling device; detecting a position, in particular 2D position or 3D position, and/or orientation of the container, in particular relative to the camera system and/or to the sample input port, in particular detecting that the container has reached a position in which the sample can be loaded into a measurement chamber by aspiring through the sample aspirator tube; detecting a type of the sample, in particular whole blood and/or plasma and/or serum and/or urine and/or a calibration solution and/or a quality control solution and/or pleural fluid and/or dialysate solution; detecting a condition and/or characteristic of the sample, in particular homogeneity and/or presence of bubbles and/or density and/or characteristic of a volume and/or characteristic of filling and/or piston position of a syringe; detecting a type of an anticoagulant, in particular by detecting a color of the sample and/or container cap and/or other recognizable features of the sample container.

Detecting the type of the container may be advantageous, since the sample aspirator tube may be required to be moved or oriented in a position and/or orientation depending on the container actually used.

Detecting the position and/or orientation of the container may also be advantageous, in order to detect a potential collision position when the sample tube or the sample aspirator tube is to be moved or oriented. Furthermore, the container may be required to be in a particular orientation and/or position in order to enable loading. When the container is in a wrong position and/or orientation, this may be indicated to the user via the optical and/or acoustical output device.

For example, in the beginning the container may be moved into the region of interest, whereupon the processor determines the type of the container as well as type and characteristics of the sample. Then, the user may be demanded to remove the container out of the region of interest. Then, the processor may control the sample aspirator positioner and move and orient the sample aspirator tube into a position and/or orientation which is compatible with the detected type of the container. Thereafter, the user may be requested or demanded, to move the open container such that the end or the tip of the sample aspirator tube is immersed into the sample, i.e. is below the sample surface. Afterwards, the user may be demanded to maintain the container in this position. Then the processor may control the sample aspirator to apply an under-pressure in order to convey or suck the sample via the lumen of the sample aspirator tube into a measurement chamber. When loading the sample is completed, the processor may control the output device to indicate to the user to remove the container. Afterwards, the processor may control the sample input port closure to close the input port. Thereafter, the processor may further control a measurement process of the sample according to the predetermined measurement procedure. After completion of the measurement, the processor may control the output device to display or indicate the measurement results to the user. In other embodiments those steps may be performed separately or in a different sequence.

Detecting the type of the sample may be advantageous in order to assess compliance of the sample with the intended measurement process. Furthermore, a characteristics of the sample may be detected, in order to decide whether the sample is suitable to be measured by the intended measurement process. If the characteristics of the sample does not comply with the intended measurement process, the user may be indicated via the output device to change the characteristics of the sample, for example to remove bubbles, to increase the homogeneity by mixing for example, or fill additional sample into the container if it is detected that the sample is of insufficient volume.

Detecting the type of the anticoagulant may also be advantageous in order to avoid erroneous measurements.

According to an embodiment of the present invention, the analysis result includes: an error related to the container and/or the sample; an incompatibility of the detected type of the container and/or the sample with an intended measurement; an incorrect positioning and/or orientation of the container.

Furthermore, the incompatibility of the detected characteristics of the sample with an intended measurement procedure may be included into the analysis results. Errors may be indicated to the user via the output device. Furthermore, any demands or requests to the user may be indicated via the output device.

Thereby, it may be enabled to correct the detected errors and then continue with further steps in the analysis of the sample. Further in case of a detected error some actions may be prohibited.

According to an embodiment of the present invention, the information indicated by the optical and/or acoustical output device, in particular displayed on the display, comprises at least one of: the image; information regarding the container and/or the sample; an error message; a warning message; a waiting message; an information message; information regarding a state of a measurement workflow.

Indicating this kind of information to the user may be advantageous, because the user then may correct the error before actually starting the measurement as such. Thereby, lost samples may be avoided as well as costs and time may be reduced.

According to an embodiment of the present invention, the information indicated by the optical and/or acoustical output device, in particular displayed on the display, comprises a demand to the user, including at least one of: indicating that the container should be moved to allow loading the sample into a measurement chamber; indicating that the container should be moved such that the sample aspirator tube is connected with the container; indicating that the container should be moved such that the sample aspirator tube is arranged in contact with the sample inside the container; indicating that the container should be removed.

Depending on the particular configuration of the input port and/or the sample aspirator and/or sample aspirator tube and the used containers, one or more other information may be indicated to the user by the output device. However, by the indications as listed above, some severe errors or problems may be reduced or even avoided which may occur conventionally during the pre-analytic phase.

According to an embodiment of the present invention, wherein to actuate the sample aspirator positioner comprises at least one of: to control the sample aspirator positioner to move the sample aspirator tube, in particular from a parking position, to a predetermined loading position, in particular being dependent on the detected type of the container; to control the sample aspirator positioner to move the sample aspirator tube, in particular from the loading position, to a parking position, in particular when the container is detected to have been removed.

In the parking position the sample aspirator tube may be retracted within the measurement apparatus or at least covered or retracted beyond the sample input port closure to be protected from damage. In the loading position the tip of the sample aspirator tube may protrude from the sample input port to be accessible to a user or at least such that a container may partly be put over the sample aspirator tube.

According to an embodiment of the present invention, to actuate the sample aspirator comprises at least one of: controlling the sample aspirator to aspirate a part of the sample into or through the sample aspirator tube, in particular depending on the detected sample and/or detected type of the container, in particular including loading at least a part of the sample from the container through the aspirator into a measurement chamber; and/or wherein to actuate the sample input port closure comprises: to control the sample input port closure to close or to open.

Thereby, loading the sample may be facilitated. Furthermore, the sample aspirator may be actuated by the processor to expel liquid or solution contained in the lumen of the sample aspirator tube out of the tip of the sample aspirator tube. Thereby also for example washing steps may be supported. Opening the sample input port may allow loading the sample, after the sample aspirator tube has been traversed out. Closing the sample input port closure may protect measurement equipment or a measurement chamber.

According to an embodiment of the present invention, to analyze the image includes, in particular in real time, detecting and/or decoding at least one label on the container and/or on a container cap, wherein the measurement support equipment is controlled further based on the detected and/or decoded at least one label.

On the container and/or on the container cap different kinds of labels may be present, e.g. printed or engraved. All information of the labels may be acquired and decoded by the processor. Thereby furthermore compliance of the sample and/or the container with the intended measurement process may be determined.

According to an embodiment of the present invention, the label comprises information regarding at least one of: a patient; a date; a user; an information regarding the sample, the label being in particular configured as at least one of: text; bar code; QR code; color coded information. The information may comprise information regarding other issues as well.

According to an embodiment of the present invention, the measurement support equipment is controlled further based on a predetermined measurement configuration and/or workflow and/or wherein to control the measurement support equipment includes to trigger and/or to perform an action or to prohibit an action and/or wherein the detection system includes at least one of: a camera system; a Laser scanning system; a RADAR-system; an infrared detection system; an ultrasonic system; wherein the camera system includes at least one 2D detector array, in particular CMOS or CCD array, sensitive for infrared and/or visible and/or ultraviolet light. Thereby, the processor may support different measurement configurations and may act depending on the measurement configuration.

According to an embodiment of the present invention, it is provided a measurement apparatus, comprising: an arrangement according to one of the preceding embodiments; a sample input port through which the sample aspirator tube is traversable; a measurement chamber adapted to be filled with the sample through a lumen of the sample aspirator tube and comprising at least one sensor unit, in particular comprising an electrode and/or optical sensor to measure at least one analyte, in particular electrolyte and/or gas and/or metabolites, in the sample.

The measurement apparatus may be arranged to analyze the liquid sample with respect to gases, electrolytes, in particular molecules or in general any interesting analytes. Examples of measurement types have been disclosed above.

According to an embodiment of the present invention, it is provided a method of supporting a measurement of a liquid sample contained in a container, the method comprising: acquiring images of a region of interest in which the container is arrangeable; analyzing at least one image of the images, in order to obtain an analysis result; and controlling measurement support equipment based on the analysis result, in particular without requiring touching anything except the container.

The measurement chamber may be fixedly connected with the sample aspirator tube and the sample aspirator positioner may be configured to move the measurement chamber for positioning the sample aspirator tube. In this embodiment, the sample aspirator tube is mechanically in a fixed manner connected with the measurement chamber. In other embodiments the sample aspirator tube is not necessarily fixedly connected to the measurement chamber. Therefore, different types of measurement chambers may be supported one having a fixedly connected loading needle and others not having fixedly attached loading equipment.

The aspects defined above and further aspects of the present invention are apparent from the examples of embodiment to be described hereinafter and are explained with reference to the examples of embodiment. The invention will be described in more detail hereinafter with reference to examples of embodiment but to which the invention is not limited.

### Brief Description of the Drawing

Figure 1 schematically illustrates a measurement apparatus according to an embodiment of the present invention comprising an arrangement for supporting a measurement of a liquid sample according to an embodiment of the present invention;
Figures 2(a) to 2(h) schematically illustrate different container types which may be detected and determined by image analysis of images taken by the arrangement for supporting a measurement of a liquid sample according to an embodiment of the present invention; and
Figures 3(a) to 3(c) illustrate images acquired by a camera system of an arrangement according to an embodiment of the present invention.

### Detailed Description

It is noted that structures or elements in different figures are labelled with reference signs differing only in the first digit. A description of one electric not explicitly described with respect to one embodiment may be taken from the description of the corresponding unit or structure or element as described with reference to another embodiment.

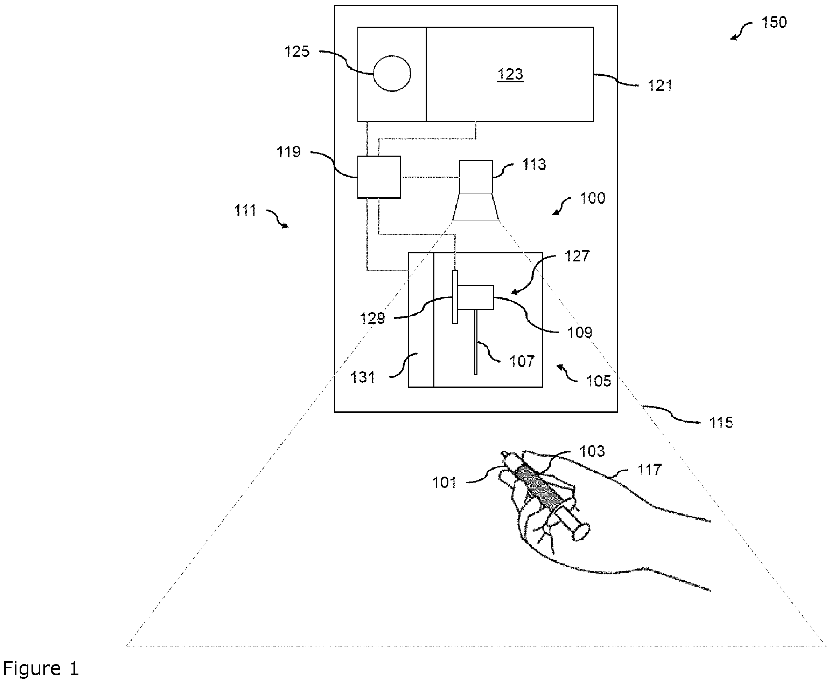

The measurement apparatus 150 schematically illustrated in **Figure 1** according to an embodiment of the present invention comprises an arrangement 100 for supporting a measurement of a liquid sample contained in a container 101 according to an embodiment of the present invention. In the illustrated embodiment, the sample 103 is contained in a syringe 101. The measurement apparatus 150 further comprises a sample input port 105 through which a sample aspirator tube 107 is traversable. In the embodiment illustrated in Figure 1 the sample aspirator tube 107 is connected to a measurement chamber 109 comprising not illustrated sensor regions for measuring the sample 103 to detect at least one analyte, for example ions or gases or metabolites within the sample 103. The measurement chamber 109 is adapted to be filled with the sample 103 through a lumen of the sample aspirator tube 107 and comprises at least one sensor unit, in particular an electrode and/or an optical sensor to measure at least one analyte.

The arrangement 100 for supporting a measurement of the liquid sample 103 contained in the container 101 comprises measurement support equipment collectively labeled with reference sign 111 which may comprise one or more elements or devices as will be explained in detail below.

The arrangement 100 further comprises a camera system 113 which is adapted to acquire images of a region of interest 115 in which the container 101 is arrangeable, in particular by a hand 117 of a user.

The arrangement 100 further comprises a processor 119 which is adapted to analyze at least one image of the images in order to obtain an analysis result and to control the measurement support equipment 111 based on the analysis result. Thereby, as is depicted in Figure 1, the processor is communicatively connected to the camera system 113 to receive digital signals representing at least one image from the camera system 113. The processor 119 analyzes at least one image, in particular plural images continuously in real time to obtain the analysis result and control the measurement support equipment 111.

The measurement support equipment 111 may comprise for example an optical and/or acoustical output device 121 for example comprising a display screen 123 and/or a loudspeaker 125. Thus, the processor can control the output device 121 depending on the analysis result which has been obtained by analyzing the images acquired by the camera system 113. By the optical and/or acoustical output device 121 different kinds of information may be indicated to a user, in order to facilitate the preparation of a measurement and ensure that the sample and/or container complies with requirements of the intended measurement process.

Additionally or alternatively, the measurement support equipment 111 comprises a sample aspirator 127, in the illustrated embodiment comprising the measurement chamber 109 as well as the aspirator tube 107. The sample aspirator further comprises not illustrated equipment for applying under-pressure or over-pressure for sucking or expelling the sample from the sample aspirator tube 107.

The measurement support equipment 111 may alternatively or additionally further comprise a sample aspirator positioner 129 which is configured to adjust the sample aspirator tube 107 in position and/or orientation. Also this sample aspirator positioner 129 may be controlled by the processor 119 based on the analysis result.

Other embodiments do not comprise a sample aspirator tube or a needle, but a sample input opening, into which e.g. a needle or a tip of a syringe may be inserted.

Further, the processor 119 may control a sample input port closure 131 based on the analysis result, to open or close the sample input port 105. The sample input port closure 131 may for example be configured as a sliding door which may slide to the right or to the left in Figure 1 for closing or opening the sample input port 105.

For loading the sample for example from a syringe, the sample aspirator tube 107 may be oriented to point out of the image plane of Figure 1, for example having an angle between the image plane of Figure 1 and the sample tube between 20° and 90°. The sample aspirator tube 107 may be brought in a position and/or orientation depending on a type of container which has been detected by analysis of the images taken by the camera system by the processor 119.

The processor 119 is configured (in particular by loaded image-processing software) to perform object detection in the images as acquired by the camera system 113.

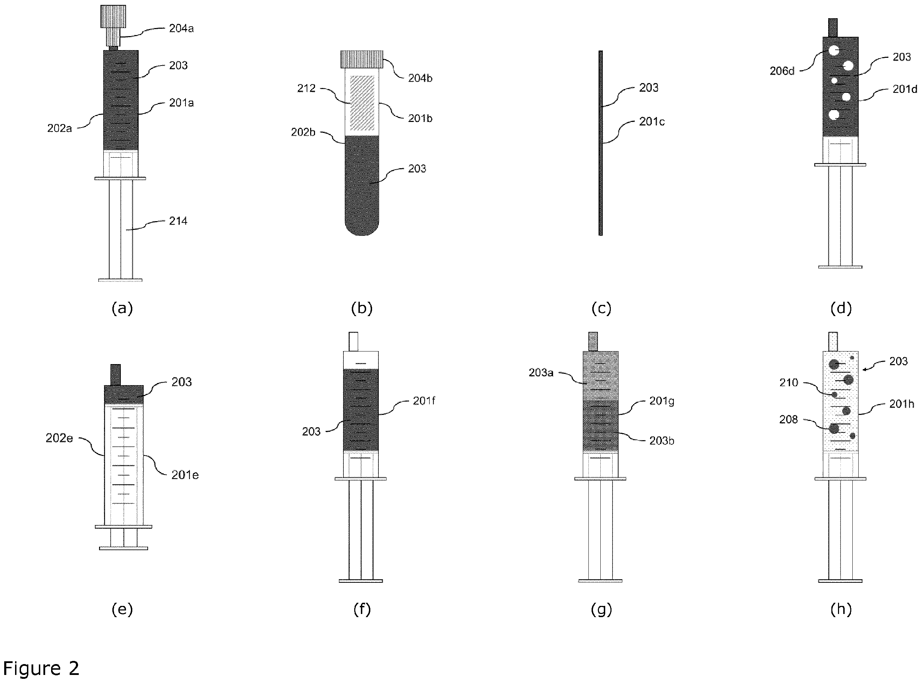

**Figures 2(a) to 2(h)** illustrate other types of containers which are detectable by the processor 119 by analyzing the image taken from the camera system 113.

The container 201a illustrated in Figure 2(a) is a syringe having a syringe body 202a, filled with sample 203 and being closed by a cap 204a and having a piston 214.

The container 201b illustrated in Figure 2(b) is a sample tube having a sample compartment 202b being closed by a cap 204b. The processor 119 may also detect a type or color or label of the cap 204b.

The container 201c illustrated in Figure 2(c) is a capillary also detectable by image detection or object detection by the processor 119.

The processor 119 not only is configured to detect different types of containers but is also configured to detect characteristics of the sample contained in one of the different types of containers. Examples are illustrated in Figure 2(d) to 2(h).

In the example illustrated in Figure 2(d), the sample comprises bubbles 206d. The sample is contained in a syringe 201d. The presence of bubbles 206d may interfere with an intended measurement process. The presence of those bubbles 206d may be indicated to a user for example by or on the optical and/or acoustical output device 121 illustrated in Figure 1.

Figure 2(e) illustrates a container showing a syringe 201e having only little amount of sample 203 filled into the syringe body 202e. The low filling level may also be detected by the processor and a warning message or an error message may be indicated to the user using for example the output device 121 illustrated in Figure 1.

Figure 2(f) illustrates a container showing a syringe 201f in which a sample is contained which is not sufficiently filled within the lumen of the syringe 201f. This low or insufficient filling may also be indicated to the user.

Figure 2(g) illustrates a container showing a syringe 201g filled with fragmented samples having a sample fraction 203a and a sample fraction 203b being of different composition. This kind of sample may also not be compatible with an intended measurement process. Therefore, this problem of fragmented sample may be indicated to the user for example using the output device 121 of the arrangement 100 for supporting a measurement.

Figure 2(h) illustrates a container showing a syringe 201h filled with sample having varying density as illustrated by different regions 208 and 210 in which the sample has different density. The problem of different density sample may also be indicated to the user using the output device 121.

The sample container 101 may comprise a label with information attached or printed directly on the outer surface of the container. An example is illustrated in the sample tube 201b in Figure 2(b), wherein a label 212 is attached to an outside of the container. The label 212 may comprise information regarding the patient, the date, the user information regarding the sample. The information may be in text form, in form of a bar-code or a QR-code, for example. Such a label 212 may in similar or different configuration also be attached or comprise in one of the other containers illustrated in Figure 2. The Information on the label is decoded by the processor 119 by analyzing images taken by camera system 113.

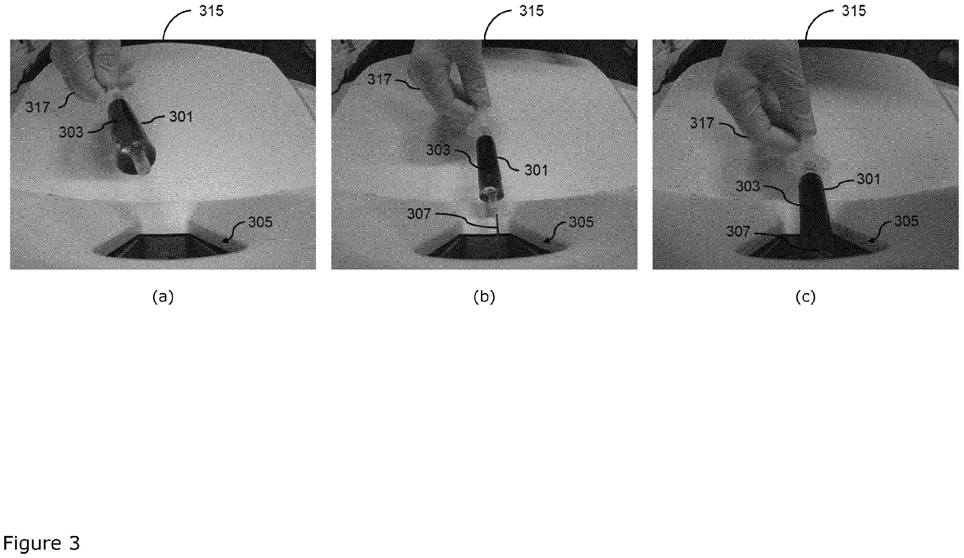

**Figure 3** illustrates images in partial images 3(a), 3(b), 3(c) as taken by the camera system 113 illustrated in Figure 1 during different steps for preparing a measurement. In the image of Figure 3(a) the container 301 (held by a user's hand 317) is moved into the region of interest 315 (in which an sample input port 305 for loading the sample is arranged) and an image is captured by the camera system 113. The processor 119 receives the image data and analyzes the image in order to detect a type of the container 301 as well as a type and characteristics of the sample 303. In the illustrated embodiment the processor correctly determines that the container is a syringe and that the sample is whole blood.

In the next step illustrated in Figure 3(b) the syringe is moved such that the sample aspirator tube 307, in particular a aspirator supply needle, is inserted through the tip opening of the syringe to be immersed into the sample.

In the next step illustrated in Figure 3(c), the syringe is further moved such that the end of the aspirator tube is fully inserted or immersed into the sample. Then the sample aspirator is controlled by the processor 119 to apply an under-pressure such that the sample is sucked into the lumen of the sample aspirator tube 307 to be conveyed to a not illustrated measurement chamber (for example similar as measurement chamber 109 illustrated in Figure 1).

Embodiments of the present invention enable a touchless control of systems in in-vitro diagnostics. Automatic identification of the user is enabled by for example acquiring the image, and analyzing for example a label on the container. Furthermore, a user tag may be recognized, for example 1D or 2D bar-code may be recognized or a QR-code may be recognized or a text on the sample may be recognized.

In other embodiments, the user may not necessarily be associated or indicated on the container or the container cap. In these embodiments, the user may identify himself by another reading process or analysis step, in which the user for example presents an ID, a bar-code in the region of interest to be imaged by the camera system and to be analyzed by the processor to extract the user ID. The patient identification may also be recognized by reading or decoding a label on the container. The patient tag may for example be represented by a 1D or 2D bar-code or a QR-code or by simple text. Furthermore, the processor may recognize the type of the sample container, the type of the sample (for example whole blood, plasma, serum, urine) and type of the anticoagulant, if applicable, in particular via color-coding.

Furthermore, the arrangement 100 may be configured to automatically identify pre-analytic errors such as (lack of) homogeneity of the sample, air-bubbles within the sample, too low sample volume, not sufficient filling, erroneous handling (for example positioning of the piston of a syringe). Furthermore, the processor may be configured to automatically recognize erroneous operation of the device, in particular the measurement apparatus 150. By indicating errors to the user, the user is enabled to correct the detected errors before conducting the measurement itself. Furthermore, embodiments of the present invention reduce the risk of a contamination of the user by portions of the sample, since the control may be performed in a touchless manner.

The camera system may comprise a wide-angle lens or a panorama lens and may comprise several cameras having respective camera lenses. When several cameras are provided for the camera system, those cameras may be positioned at different positions, such that different viewing angles are realized to image the respective container from different viewing angles. Thereby a three-dimensional reconstruction of the container may be possible also simplifying the detection of the type of the container as well as the detection of the sample characteristics. Optionally, an illumination source may be provided for appropriately illuminating the region of interest including the container.

Object detection and recognition may employ detection, tracking and classification of objects, for example by deep learning supported computer vision system. The result may for example be an object which may be associated to a predefined class of objects and which at least two-dimensional position may be known. Those kinds of information may be utilized to extract particular objects from the image and in order to examine the sample container regarding the above described properties.

The position of the detected and classified object (for example type of container) may then be utilized for touchless controlling of the measurement support equipment. Thereby, an interface may be employed which may take over the control over the measurement workflow. Device actions may be triggered upon recognition of a predefined sample container or those device actions may be interrupted or prohibited. In combination with the information which may be extracted from the object, different actions may be distinguished and situations may be assessed.

For example, the Figure 3(a) to 3(c) illustrates different steps of an exemplary preparation workflow. In Figure 3(a) the sample container is presented for classification. Furthermore, patient data or other relevant information are read-in from the sample container. Then a particular action is triggered in that the sample input is moved into a position corresponding to the detected type of the sample container.

In Figure 3(b) the syringe is attached to the sample needle. In Figure 3(c) the state that the syringe is attached to the needle is recognized by the system and thereupon the sample is aspirated. The removing of the sample container may correspond to the reversed sequence of images 3(c), 3(b), 3(a).

Embodiments of the present invention may enable an automatic identification of user, patient, type of container, type or probe, type of anticoagulant. Furthermore, embodiments provide a method and an apparatus for automatically identifying or pre-analytic errors for example homogeneity of the sample.

## Claims

1. An arrangement (100) for supporting a measurement of a liquid sample (103) contained in a container (101), the arrangement comprising:
a measurement support equipment (111, 121, 127);
a detection system adapted to acquire object information of an object in a region of interest (115) in which the container is arrangeable; and
a processor (119) adapted:
to analyze the object information, in order to obtain an analysis result; and
to control the measurement support equipment (111, 121, 127) based on the analysis result,
wherein the measurement support equipment is in particular controlled without requiring touching anything except the container.

2. Arrangement according to the preceding claim, wherein the detection system comprises:
a camera system (113) adapted to acquire images of the region of interest (115) as at least a part of the object information,
wherein the processor (119) is adapted to analyze at least one image of the images, in order to obtain at least partly the analysis result.

3. Arrangement according to one of the preceding claims, wherein the measurement support equipment comprises at least one of:
an optical and/or acoustical output device (121), in particular a display (123) ;
a sample input system adapted to allow input of the sample for the measurement, the sample input system in particular including at least one of:
a sample input opening;
a sample aspirator (127) including a sample aspirator tube (107), in particular needle, having a lumen through which the sample can be conveyed towards a measurement chamber (109);
a sample aspirator positioner (129) configured to adjust the sample aspirator tube (107) in position and/or orientation;
a sample input port closure (131) configured to open and close an input port (105) through which the sample aspirator tube is traversable to be accessible in the region of interest (115).

4. Arrangement according to one of the preceding claims, wherein to control the measurement support equipment based on the analysis result comprises at least one of:
to indicate information on the optical and/or acoustical output device (121), in particular to display information on the display (123);
to control the sample input system;
to actuate the sample aspirator (127);
to actuate the sample aspirator positioner (129);
to actuate the sample input port closure (131).

5. Arrangement according to one of the preceding claims 2 to 4, wherein to analyze the at least one image includes, in particular in real time:
object detection, in particular using methods of unsupervised and/or supervised machine learning configured to identify in the image at least one object type from a group of candidate object types;
wherein the measurement support equipment is controlled based on the identified object type.

6. Arrangement according to one of the preceding claims 2 to 5, wherein to analyze the at least one image includes, in particular in real time, at least one of:
detecting a type of the container (101), in particular syringe (201a) or sample tube (201b) or capillary (201c) or sampling device;
detecting a position, in particular 2D position or 3D position, and/or orientation of the container (101), in particular relative to the camera system and/or to the sample input port, in particular detecting that the container has reached a position in which the sample can be loaded into a measurement chamber by sucking through the sample aspirator tube;
detecting a type of the sample (103), in particular whole blood and/or plasma and/or serum and/or urine and/or a calibration solution and/or a quality control solution and/or pleural fluid and/or dialysate solution;
detecting a condition and/or characteristic of the sample, in particular homogeneity and/or presence of bubbles (206d) and/or density and/or characteristic of a volume and/or characteristic of filling and/or piston position of a syringe;
detecting a type of an anticoagulant, in particular by detecting a color of the sample and/or container cap (204a, 204b) and/or other recognizable features of the sample container.

7. Arrangement according to one of the preceding claims, wherein the analysis result includes:
an error related to the container and/or the sample;
an incompatibility of the detected type of the container and/or the sample with an intended measurement;
an incorrect positioning and/or orientation of the container.

8. Arrangement according to one of the preceding claims, wherein the information indicated by the optical and/or acoustical output device (121), in particular displayed on the display, comprises at least one of:
the image;
information regarding the container and/or the sample;
an error message;
a warning message;
a waiting message;
an information message;
information regarding a state of a measurement workflow.

9. Arrangement according to one of the preceding claims, wherein the information indicated by the optical and/or acoustical output device (121), in particular displayed on the display (123), comprises a demand to the user, including at least one of:
indicating that the container should be moved to allow loading the sample into a measurement chamber;
indicating that the container should by moved such that the sample aspirator tube is connected with the container;
indicating that the container should be moved such that the sample aspirator tube is arranged in contact with the sample inside the container;
indicating that the container should be removed.

10. Arrangement according to one of the preceding claims, wherein to actuate the sample aspirator positioner (129) comprises at least one of:
to control the sample aspirator positioner to move the sample aspirator tube (107), in particular from a parking position, to a predetermined loading position, in particular being dependent on the detected type of the container;
to control the sample aspirator positioner to move the sample aspirator tube (107), in particular from the loading position, to a parking position, in particular when the container is detected to have been removed.

11. Arrangement according to one of the preceding claims,
wherein to actuate the sample aspirator (127) comprises at least one of:
controlling the sample aspirator to aspirate a part of the sample (103) into or through the sample aspirator tube (107), in particular depending on the detected sample and/or detected type of the container, in particular including loading at least a part of the sample from the container through the aspirator into a measurement chamber; and/or
wherein to actuate the sample input port closure (131) comprises:
to control the sample input port closure to close or to open.

12. Arrangement according to one of the preceding claims 2 to 11,
wherein to analyze the image includes, in particular in real time, detecting and/or decoding at least one label (212) on the container and/or on a container cap,
wherein the measurement support equipment is controlled further based on the detected and/or decoded at least one label, and/or
wherein the label (212) comprises information regarding at least one of:
a patient;
a date;
a user;
an information regarding the sample,
the label being in particular configured as at least one of:
text;
bar code;
QR code;
color coded information.

13. Arrangement according to one of the preceding claims,
wherein the measurement support equipment is controlled further based on a predetermined measurement configuration and/or workflow and/or
wherein to control the measurement support equipment includes to trigger and/or to perform an action or to prohibit an action and/or
wherein the detection system includes at least one of: a camera system; a Laser scanning system; a RADAR-system; an infrared detection system; an ultrasonic system;
wherein the camera system (113) includes at least one 2D detector array, in particular CMOS or CCD array, sensitive for infrared and/or visible and/or ultraviolet light.

14. Measurement apparatus (150), comprising:
an arrangement (100) according to one of the preceding claims;
a sample input port (105) through which the sample aspirator tube (107) is traversable;
a measurement chamber (109) adapted to be filled with the sample through a lumen of the sample aspirator tube (107) and comprising at least one sensor unit, in particular comprising an electrode and/or optical sensor to measure at least one analyte, in particular electrolyte and/or gas and/or metabolites, in the sample.

15. Method of supporting a measurement of a liquid sample (103) contained in a container (101), the method comprising:
acquiring images of a region of interest (115) in which the container (101) is arrangeable;
analyzing at least one image of the images, in order to obtain an analysis result; and
controlling measurement support equipment (111, 121, 127) based on the analysis result, in particular without requiring touching anything except the container.
